(19) Europäisches Patentamt
European Patent Office
Office européen des brevets

(11) Veröffentlichungsnummer: **0 176 026**
**B1**

(12) # EUROPÄISCHE PATENTSCHRIFT

(45) Veröffentlichungstag der Patentschrift: **25.11.87**

(51) Int. Cl.[4]: **C 07 C 63/70,** C 07 C 51/00

(21) Anmeldenummer: **85111789.5**

(22) Anmeldetag: **18.09.85**

(54) **Verfahren zur Herstellung von 2,4-Dichlor-5-fluor-benzoesäure.**

(30) Priorität: **27.09.84 DE 3435392**

(43) Veröffentlichungstag der Anmeldung: **02.04.86 Patentblatt 86/14**

(45) Bekanntmachung des Hinweises auf die Patenterteilung: **25.11.87 Patentblatt 87/48**

(84) Benannte Vertragsstaaten: **BE CH DE FR GB IT LI NL**

(56) Entgegenhaltungen:
**EP-A-0 048 375**
**DE-A-2 412 855**

**INTERSCIENCE PUBLISHERS, New York-London-Sydney, III Acylation and Related Reactions, Part 1 G.A. OLAH "Friedel-Crafts and related reactions" Seite 166**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Roeberstrasse 8, D-5600 Wuppertal 1 (DE)**
Erfinder: **Grohe, Klaus, Dr., Am Wasserturm 10, D-5068 Odenthal (DE)**

**EP 0 176 026 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein neues Verfahren zur Herstellung von 2,4-Dichlor-5-fluor-benzoesäure, einem Zwischenprodukt für die Herstellung von antibakteriellen Mitteln.

Es ist bereits bekannt, daß Trihalogenbenzoesäuren durch Verseifung von Trihalogen-trihalogenmethylbenzol hergestellt werden können. So wird bei der Verseifung von 2,4-Dichlor-5-fluor-trichlormethylbenzol, 2,4-Dichlor-5-fluor-benzoesäure gebildet (EP-OS 78 362).

Weiterhin ist bekannt, daß die Acylierung mit aliphatischen Carbonsäurehalogeniden bei Dihalogenbenzolen sehr schwierig ist und bei Trihalogenbenzolen nicht stattfinden soll (vgl. Methoden der organischen Chemie (Houben-Weyl-Müller) Band 7/2a, 43 (1973), Thieme-Verlag, Stuttgart).

Außerdem ist bekannt, daß bei der Acylierung mit Carbonsäureanhydriden, wie z. B. Essigsäureanhydrid, aus 2,4-Dichlorfluorbenzol, in Gegenwart von Aluminiumtrichlorid, in schlechter Ausbeute 2,4-Dichlor-5-fluor-acetophenon erhalten wird (vgl. CA 58, 11243 g).

Überraschenderweise wurde nun gefunden, daß man 2,4-Dichlor-5-fluorbenzoesäure der Formel (I)

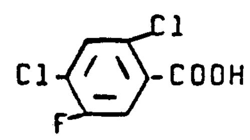

(I)

in sehr guter Ausbeute und hoher Reinheit erhält, wenn man 2,4-Dichlor-fluorbenzol mit Acetylchlorid, in Gegenwart von Acylierungskatalysatoren und gegebenenfalls in Gegenwart von Verdünnungsmitteln, bei Temperaturen zwischen 10° C und 150° C umsetzt und das auf diese Weise entstandene Reaktionsprodukt 2,4-Dichlor-5-fluor-acetophenon der Formel (II)

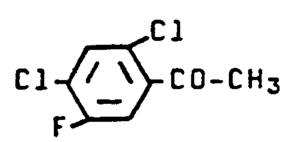

(II)

gegebenenfalls nach Isolierung, mit einer Natriumhypochloritlösung (in Form der sogenannten Chlorlauge), bei einer Temperatur zwischen 0° C und 140° C umsetzt.

Überraschenderweise gelingt es, mit Hilfe des erfindungsgemäßen Verfahrens eine Acylierung von 2,4-Dichlorfluorbenzol mit einem Carbonsäurechlorid in meta-Stellung zum elektronegativeren Halogen in hoher Ausbeute und Selektivität durchzuführen. Dies ist umso überraschender, als nach dem Stand der Technik bei der Acylierung von Halogenbenzolen die Acylierung in ortho- oder para-Stellung vom elektronegativeren Halogen hätte erwartet werden müssen (vergl. Methoden der organischen Chemie (Houben-Weyl-Müller) Band 7/2a 43 (1973), Thieme-Verlag, Stuttgart).

Die oben genannten bekannten Verfahren besitzen eine Reihe von Nachteilen. So wird bei der Herstellung von 2,4-Dichlor-5-fluor-trichlormethylbenzol als Zwischenprodukt ein Triazen hergestellt, das sich wegen seiner ungünstigen physiologischen Eigenschaften nur schwierig handhaben läßt.

Weiterhin sind nach diesem Verfahren für die Herstellung von 2,4-Dichlor-5-fluor-benzoesäure mehrere Stufen erforderlich.

Bei der in der Literatur beschriebenen Acylierung von 2,4-Dichlor-5-fluorbenzol in Gegenwart von Essigsäureanhydrid ist die Ausbeute an Reaktionsprodukt sehr gering.

Verwendet man 2,4-Dichlor-fluorbenzol und Acetylchlorid als Ausgangsstoffe, Aluminiumchlorid als Katalysator und Chlorlauge, so kann der Reaktionsverlauf durch das folgende Formelschema wiedergegeben werden:

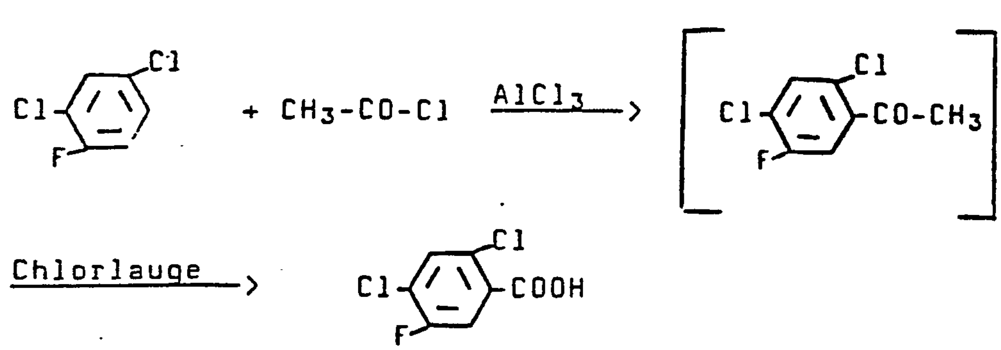


2,4-Dichlor-fluorbenzol ist eine bekannte Verbindung der organischen Chemie.

Die Reaktionstemperatur kann in einem größeren Bereich variiert werden. So arbeitet man bei der Acylierung erfindungsgemäss bei Temperaturen zwischen 10°C und 150°C, vorzugsweise zwischen 20°C und

130° C, insbesondere zwischen 80° C und 130° C. Die anschließende Oxidation mit der sogenannten Chlorlauge wird im allgemeinen bei Temperaturen zwischen 0° C und 140° C, vorzugsweise zwischen 20° C und 120° C durchgeführt. Die Reaktion wird im allgemeinen bei Normaldruck durchgeführt.

Das erfindungsgemäße Verfahren wird vorzugsweise ohne Verdünnungsmittel durchgeführt.

Als Katalysator kommen für das erfindungsgemäße Verfahren Acylierungskatalysatoren in Frage wie z. B. Eisen(III)-chlorid, Zinkchlorid oder Aluminiumchlorid, vorzugsweise Aluminiumchlorid.

Als Oxidationsmittel kommt für das erfindungsgemäße Verfahren die sogenannte Chlorlauge, eine Natriumhypochloritlösung in Wasser in Betracht.

Bei der Durchführung des erfindungsgemäßen Verfahrens setzt man im allgemeinen auf 1 Mol 2,4-Dichlorfluorbenzol, 1 bis 3 Mol Acetylchlorid und 1 bis 3 Mol Aluminiumchlorid ein. Nach beendeter Reaktion wird das Reaktionsgemisch auf Eis gegeben und in einem mit Wasser nicht mischbaren Verdünnungsmittel, wie z. B. Methylenchlorid oder Chloroform, aufgenommen. Das Reaktionsprodukt kann aber auch ohne Verwendung von Verdünnungsmitteln abgetrennt werden. Gegebenenfalls nach Entfernen des Extraktionsmittel wird der Rückstand in Gegenwart von 2 bis 4 l, vorzugsweise 2,1 bis 3,3 l Chlorlauge (150 g aktives Chlor/l) pro Mol Ausgangsprodukt oxidiert. Anschließend wird die 2,4-Dichlor-5-fluor-benzolsäure mit einer Mineralsäure, wie z. B. Salzsäure, ausgefällt und dann abgesaugt.

Die nach dem erfindungsgemäßen Verfahren leicht zugängliche 2,4-Dichlor-5-fluor-benzoesäure läßt sich beispielsweise zur Synthese von antibakteriellen Mitteln einsetzen. So lassen sich aus dieser Säure z. B. gemäß folgenden Gleichungen, die substituierten Oxochinolincarbonsäuren, Verbindungen mit hoher bakteriziden Potenz, herstellen (vgl. z. B. EP-OS 78 362):

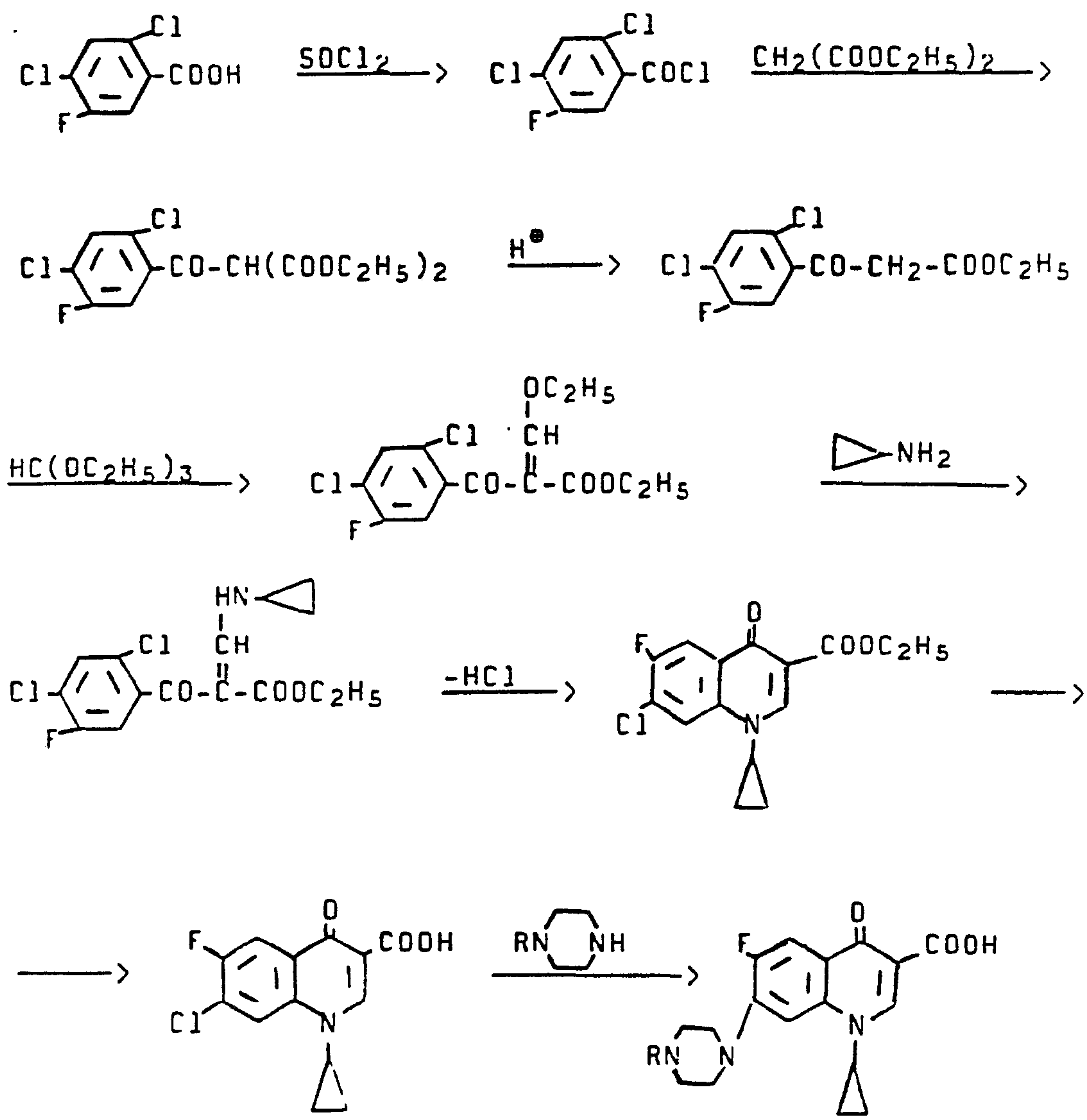


R = H, Alkyl

**Herstellungsbeispiel**

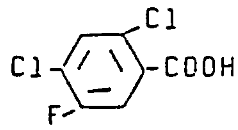

(I)

Eine Mischung aus 33 g (0,2 Mol) 2,4-Dichlor-fluorbenzol und 66,8 g (0,5 Mol) Aluminiumchlorid wird bei 20°C bis 40°C mit 23,6 g (0,3 Mol) Acetylchlorid versetzt und dann 2 Stunden bei 120°C gerührt. Die noch heiße Mischung wird auf 250 g Eis gegeben und das sich abscheidende Öl in Methylenchlorid aufgenommen. Das Lösungsmittel wird abgedampft und der Rückstand wird mit 450 ml Chlorlauge (150 g aktives Chlor/l) versetzt und erst 1 Stunde ohne Heizung gerührt und dann 2 Stunden unter Rückfluß gekocht. Nach Abtrennen des entstandenen Chloroforms gibt man 300 ml Wasser zu, versetzt mit 10 ml 40 %iger Natriumhydrogensulfit-Lösung und anschließend mit konzentrierter Salzsäure bis ein pH-Wert 1 erreicht wird.

Man erhält auf diese Weise 33,5 g (80 % der Theorie) 2,4-Dichlor-5-fluor-benzoesäure als farbloses Pulver vom Schmelzpunkt 139°C.

**Patentansprüche**

1. Verfahren zur Herstellung von 2,4-Dichlor-5-fluor-benzoesäure der Formel (I)

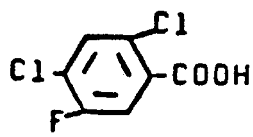

(I)

dadurch gekennzeichnet, daß man 2,4-Dichlor-fluor-benzol mit Acetylchlorid, in Gegenwart von Acylierungskatalysatoren und gegebenenfalls in Gegenwart von Verdünnungsmitteln, bei Temperaturen zwischen 10°C und 150°C umsetzt und das auf diese Weise entstandene Reaktionsprodukt 2,4-Dichlor-5-fluor-acetophenon der Formel (II)

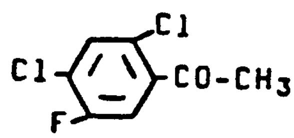

(II)

gegebenenfalls nach Isolierung, mit einer Natriumhypochloritlösung (in Form der sogenannten Chlorlauge), bei einer Temperatur zwischen 0°C und 140°C umsetzt und die Verbindung der Formel (I) nach üblichen Methoden isoliert.

2. Verfahren gemäß Anspruch 1, dadurch gekennzeichnet, daß man als Acylierungskatalysator Aluminiumtrichlorid verwendet.

3. Verfahren gemäß den Ansprüchen 1 und 2, dadurch gekennzeichnet, daß man die Umsetzung mit Acetylchlorid bei Temperaturen zwischen 20°C und 130°C durchführt.

4. Verfahren gemäß Ansprüchen 1 und 2, dadurch gekennzeichnet, daß man die Umsetzung mit der Natriumhypochloritlösung (Chlorlauge) bei Temperaturen zwischen 20°C und 120°C durchführt.

5. Verfahren gemäß Ansprüchen 1 bis 4, dadurch gekennzeichnet, daß das im Verlaut des Verfahrens entstandene 2,4-Dichlor-5-fluor-acetophenon ohne Isolierung mit der Natriumhypochloritlösung (Chlorlauge) umgesetzt wird.

**Claims**

1. Process for the preparation of 2,4-dichloro-5-fluoro-benzoic acid of the formula (I)

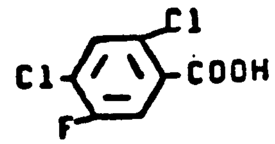
(I)

characterised in that 2,4-dichlorofluorobenzene is reacted with acetyl chloride, in the presence of acylation catalysts and optionally in the presence of diluents, at temperatures between 10° C and 150° C and the reaction product, 2,4-dichloro-5-fluoro-acetophenone of the formula (II)

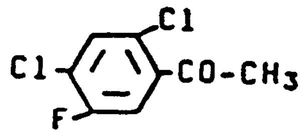
(II)

formed in this way is, if appropriate after isolation, reacted with a sodium hypochlorite solution (in the form of the so-called chlorinated soda solution) at a temperature between 0° C and 140° C and the compound of the formula (I) is isolated by conventional methods.

2. Process according to Claim 1, characterised in that aluminium trichloride is used as the acylation catalyst.

3. Process according to Claims 1 and 2, characterised in that the reaction with acetyl chloride is carried out at temperatures between 20° C and 130° C.

4. Process according to Claims 1 and 2, characterised in that the reaction with the sodium hypochlorite solution (chlorinated soda solution) is carried out at temperatures between 20° C and 120° C.

5. Process according to Claims 1 to 4, characterised in that the 2,4-dichloro-5-fluoro-acetophenone formed during the course of the process is, without isolation, reacted with the sodium hypochlorite solution (chlorinated soda solution).

**Revendications**

1. Procédé de production d'acide 2,4-dichloro-5-fluorobenzoïque de formule (I)

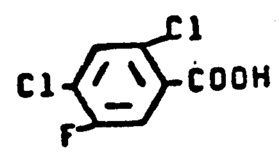
(I)

caractérisé en ce qu'on fait réagir le 2,4-dichlorofluoro-benzène avec le chlorure d'acétyle en présence de catalyseurs d'acylation et, le cas échéant, en présence de diluants, à des températures comprises entre 10 et 150° C et on fait réagir le produit réactionnel obtenu de cette façon, à savoir la 2,4-dichloro-5-fluoracétophénone de formule (II)

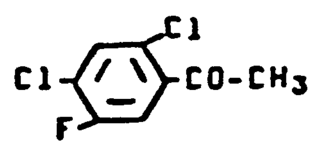
(II)

le cas échéant après isolement, avec une solution d'hypochlorite de sodium (sous la forme de liqueur de blanchiment) à une température comprise entre 0 et 140° C et on isole le composé de formule (I) par des procédés classiques.

2. Procédé suivant la revendication 1, caractérisé en ce qu'on utilise comme catalyseur d'acylation le trichlorure d'aluminium.

3. Procédé suivant les revendications 1 et 2, caractérisé en ce qu'on conduit la réaction avec le chlorure d'acétyle à des températures comprises entre 20 et 130° C.

4. Procédé suivant les revendications 1 et 2, caractérisé en ce qu'on conduit la réaction avec la solution d'hypochlorite de sodium (liqueur de blanchiment) à des températures comprises entre 20 et 120° C.

5. Procédé suivant les revendications 1 à 4, caractérisé en ce que la 2,4-dichloro-5-fluoracétophénone produite au cours du procédé est amenée à réagir sans isolement avec la solution d'hypochlorite de sodium (liqueur de blanchiment).